# EUROPEAN PATENT APPLICATION

(11) **EP 4 357 366 A1**
(43) Date of publication of application: **24.04.2024**
(21) Application number: 22824305.1
(22) Date of filing: 17.06.2022
(51) Int. Cl.: C07K 16/46, C07K 16/18, C07K 19/00, A61K 39/00, A61P 35/00

(54) **BISPECIFIC FUSION PROTEIN**

(30) Priority: 18.06.2021 CN 202110676229
(71) Applicant: Hangzhou Sumgen Biotech Co., Ltd., Hangzhou, Zhejiang 310056 (CN); Sumgen Mab (Beijing) Biotech Co., Ltd., Beijing 100176 (CN)
(72) Inventor: LV, Ming, Hangzhou, Zhejiang 310052 (CN); DING, Xiaoran, Hangzhou, Zhejiang 310052 (CN); MIAO, Shiwei, Hangzhou, Zhejiang 310052 (CN); TAN, Bin, Hangzhou, Zhejiang 310052 (CN)
(74) Representative: Weickmann & Weickmann PartmbB
(86) International application number: PCT/CN2022/099322
(87) International publication number: WO 2022/262832

(57) **Abstract**

Provided is a bispecific fusion protein, comprising a first binding domain and a second binding domain. The first binding domain comprises an antibody specifically binding to PD-L1, the antibody comprises two antibody light chains and two antibody heavy chains, and the antibody light chains and the antibody heavy chains are linked by means of disulfide bonds. The second binding domain comprises an Ig-like domain of VEGFR1 and an Ig-like domain of VEGFR2; the N-terminus of the Ig-like domain of VEGFR1 or the N-terminus of the Ig-like domain of VEGFR2 is directly or indirectly linked to the C-terminus of the antibody heavy chains, separately. A pharmaceutical composition comprising the bispecific fusion protein and a pharmaceutical use thereof are further provided. The bispecific fusion protein can effectively and safely kill tumor cells.

## Description

### FIELD OF THE INTVENTION

The present application relates to the field of biomedicine, and in particular, relates to a bispecific fusion protein.

### BACKGROUND OF THE INVENTION

At present, tumor diseases are becoming increasingly serious, and immune checkpoints PD-1/PD-L1 and VEGF have become important targets for tumor treatment.

PD-L1 is overexpressed in a variety of malignant tumors and is often associated with poor prognosis. The therapeutic-targeting of PD-1 and other molecules (for example, PD-L1 and PD-L2) transmitting signals by their interaction with PD-1 is a popular field for tumor treatment.

VEGF is a powerful and unique mitogen of a vascular endothelial cell, and can promote all aspects of the angiogenesis process. The role of each VEGF receptor (VEGFR) varies. VEGFR-2 plays a role in regulation of endothelial growth, differentiation, and permeability; and VEGFR-1 is involved in the regulation of endothelial cell migration and aggregation, and inhibits signal transmission by means of VEGFR-2. VEGF binds to VEGFR for dimerization and autophosphorylation of VEGFR, and transmits signals through a plurality of intracellular pathways to ultimately come into play.

There is an urgent need for a novel fusion protein capable of effectively suppressing tumor growth.

### SUMMARY OF THE INVENTION

The present application provides a bispecific fusion protein, and its corresponding polynucleotide, vector, cell, preparation method, pharmaceutical composition, and use. The bispecific fusion protein of the present application has at least one of the following properties: 1) specifically binding to PD-L1; 2) blocking the binding of PD-1 to PD-L1; 3) specifically binding to VEGF; 4) blocking the binding of VEGF to VEGFR; 5) inhibiting (in vivo) tumor growth; 6) activating immune cells (for example, T cells); 7) promoting the secretion of cytokines (for example, interferons and/or cytokines) by immune cells (for example, T cells); and/or, 8) inhibiting tumor neovascularization and vascular normalization.

In one aspect, the present application provides a bispecific fusion protein, comprising a first binding domain and a second binding domain, wherein: the first binding domain comprises an antibody specifically binding to PD-L1, wherein the antibody comprises two antibody light chains and two antibody heavy chains, and the antibody light chains and the antibody heavy chains are linked via disulfide bonds; the second binding domain comprises an Ig-like domain of VEGFR1 and an Ig-like domain of VEGFR2; wherein an N-terminus of the Ig-like domain of VEGFR1 or an N-terminus of the Ig-like domain of VEGFR2 is directly or indirectly linked to C-termini of the antibody heavy chains, respectively.

In some embodiments, the first binding domain specifically binds to human PD-L1.

In some embodiments, the second binding domain specifically binds to a human VEGF family.

In some embodiments, the second binding domain specifically binds to a protein selected from the group consisting of: VEGF-A, VEGF-B, VEGF-C, VEGF-D, and PLGF.

In some embodiments, the Ig-like domain of VEGFR1 comprises an amino acid sequence as set forth in SEQ ID NO.: 1.

In some embodiments, the Ig-like domain of VEGFR2 comprises an amino acid sequence as set forth in SEQ ID NO.: 2.

In some embodiments, the Ig-like domain of VEGFR1 and the Ig-like domain of VEGFR2 are directly linked.

In some embodiments, the second binding domain comprises an amino acid sequence as set forth in SEQ ID NO : 28.

In some embodiments, the indirect linking is conducted via a linker.

In some embodiments, the linker comprises an amino acid sequence as set forth in SEQ ID NO.: 3.

In some embodiments, the antibody light chains each comprise a light chain variable region, which comprises LCDRs 1-3, with the LCDR1 comprising an amino acid sequence as set forth in any one of SEQ ID NOs.: 4 and 10.

In some embodiments, the LCDR2 comprises an amino acid sequence as set forth in any one of SEQ ID NOs.: 5 and 11.

In some embodiments, the LCDR3 comprises an amino acid sequence as set forth in any one of SEQ ID NOs.: 6 and 12.

In some embodiments, amino acid sequences of the LCDRs 1-3 are selected from any one of the following groups:
(1) the LCDR1 comprises an amino acid sequence as set forth in SEQ ID NO.: 4, the LCDR2 comprises an amino acid sequence as set forth in SEQ ID NO.: 5, and the LCDR3 comprises an amino acid sequence as set forth in SEQ ID NO.: 6; and
(2) the LCDR1 comprises an amino acid sequence as set forth in SEQ ID NO.: 10, the LCDR2 comprises an amino acid sequence as set forth in SEQ ID NO.: 11, and the LCDR3 comprises an amino acid sequence as set forth in SEQ ID NO.: 12.

In some embodiments, the antibody light chains each comprise a light chain variable region, which comprises an amino acid sequence as set forth in any one of SEQ ID NOs.: 16-17.

In some embodiments, the antibody light chains each comprise a light chain constant region, which is derived from a light chain constant region of a protein selected from the group consisting of: Igx and Igλ.

In some embodiments, the antibody heavy chains each comprise a heavy chain variable region, which comprises HCDRs 1-3, with the HCDR1 comprising an amino acid sequence as set forth in any one of SEQ ID NOs.: 7 and 13.

In some embodiments, the HCDR2 comprises an amino acid sequence as set forth in any one of SEQ ID NOs.: 8 and 14.

In some embodiments, the HCDR3 comprises an amino acid sequence as set forth in any one of SEQ ID NOs.: 9 and 15.

In some embodiments, the HCDRs 1-3 comprise amino acid sequences selected from the following groups:
(1) the HCDR1 comprises an amino acid sequence as set forth in SEQ ID NO.: 7, the HCDR2 comprises an amino acid sequence as set forth in SEQ ID NO.: 8, and the HCDR3 comprises an amino acid sequence as set forth in SEQ ID NO.: 9; and
(2) the HCDR1 comprises an amino acid sequence as set forth in SEQ ID NO.: 13, the HCDR2 comprises an amino acid sequence as set forth in SEQ ID NO.: 14, and the HCDR3 comprises an amino acid sequence as set forth in SEQ ID NO.: 15.

In some embodiments, the antibody heavy chains each comprise a heavy chain constant region, which comprises an amino acid sequence as set forth in any one of SEQ ID NOs.: 18-19.

In some embodiments, the antibody heavy chains each comprises an Fc region.

In some embodiments, the Fc region is derived from an Fc of a protein selected from the group consisting of: IgG1 and IgG4.

In some embodiments, the Fc region comprises an amino acid mutation at an amino acid position selected from the group consisting of: N298, D357 and L359.

In some embodiments, the Fc region comprises an amino acid mutation selected from the group consisting of: N298A, D357E and L359M.

In some embodiments, the Fc region comprises an amino acid sequence as set forth in any one of SEQ ID NOs.: 20-22.

In some embodiments, the bispecific fusion protein is a multimer consisting of two copies of first polypeptide chain and second polypeptide chain, wherein the first polypeptide chain comprises the antibody light chains, and wherein the second polypeptide chain comprises the antibody heavy chains, the linker and the second binding domain in sequence from an N-terminus.

In some embodiments, the first polypeptide chain comprises an amino acid sequence as set forth in any one of SEQ ID NOs.: 23-24.

In some embodiments, the second polypeptide chain comprises an amino acid sequence as set forth in any one of SEQ ID NOs.: 25-27.

In some embodiments, the first polypeptide chain comprises an amino acid sequence as set forth in SEQ ID NO.: 23, and the second polypeptide chain comprises an amino acid sequence as set forth in SEQ ID NO.: 25; or, wherein the first polypeptide chain comprises an amino acid sequence as set forth in SEQ ID NO.: 24, and the second polypeptide chain comprises an amino acid sequence as set forth in SEQ ID NO.: 26; or, wherein the first polypeptide chain comprises an amino acid sequence as set forth in SEQ ID NO.: 23, and the second polypeptide chain comprises an amino acid sequence as set forth in SEQ ID NO.: 27.

In another aspect, the present application provides a polynucleotide encoding the bispecific fusion protein of the present application.

In another aspect, the present application provides a vector, comprising the polynucleotide of the present application.

In another aspect, the present application provides a cell, comprising the vector of the present application.

In another aspect, the present application provides a method for preparing the bispecific fusion protein of the present application, comprising culturing the cell of the present application under a condition suitable for expressing the bispecific fusion protein of the present application.

In another aspect, the present application provides a pharmaceutical composition, comprising the bispecific fusion protein of the present application and an optionally pharmaceutically acceptable carrier.

In another aspect, the present application provides a pharmaceutical molecule, comprising the bispecific fusion protein of the present application.

In another aspect, the present application provides use of the bispecific fusion protein, the polynucleotide, the vector, the cell, the pharmaceutical composition, and/or the pharmaceutical molecule of the present application in preparation of a medicament for treating diseases, wherein the diseases comprise tumors.

In some embodiments, the diseases comprise solid tumors and non-solid tumors.

In some embodiments, the diseases comprise PD-L1-positive tumors.

In some embodiments, the tumors comprise a lung cancer, a colorectal cancer, a cervical cancer, a liver cancer, a gastric cancer and/or a renal cancer.

In another aspect, the present application provides a method for inhibiting angiogenesis, comprising administering an effective amount of the bispecific fusion protein, the polynucleotide, the vector, the cell, the pharmaceutical composition, and/or the pharmaceutical molecule of the present application.

In another aspect, the present application provides a method for inhibiting an activity of a VEGF receptor ligand, comprising administering an effective amount of the bispecific fusion protein, the polynucleotide, the vector, the cell, the pharmaceutical composition, and/or the pharmaceutical molecule of the present application.

In another aspect, the present application provides a method for inhibiting of PD-L1 activity, comprising administering an effective amount of the bispecific fusion protein, the polynucleotide, the vector, the cell, the pharmaceutical composition, and/or the pharmaceutical molecule of the present application.

Other aspects and advantages of the present application can be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the invention involved in the present application are listed in the appended claims. The characteristics and advantages of the invention involved in the present application may be better understood by referring to the exemplary embodiments and the accompanying drawings described in detail below. The accompanying drawings are briefly illustrated as follows.
FIG. 1 shows the structure of a bispecific fusion protein according to the present application;
FIG. 2 shows the specific binding of the bispecific fusion protein according to the present application to human PD-L1;
FIG. 3 shows the specific binding of the bispecific fusion protein according to the present application to VEGF165;
FIG. 4 shows the simultaneous specific binding of the bispecific fusion protein according to the present application to PD-L1 and VEGF165;
FIG. 5 shows the blocking of the interaction between PD-1 and PD-L1 by the bispecific fusion protein according to the present application;
FIG. 6 shows the blocking of the interaction between VEGF and VEGFR by the bispecific fusion protein according to the present application;
FIG. 7 shows the blocking of the interaction between VEGF 165 and VEGFR2 by the bispecific fusion protein according to the present application;
FIG. 8 shows the blocking of the interaction between VEGF121 and VEGFR2 by the bispecific fusion protein according to the present application;
FIG. 9 shows the blocking of the interaction between VEGF C and VEGFR2 by the bispecific fusion protein according to the present application;
FIG. 10 shows the inhibition of HUVEC cell proliferation by the bispecific fusion protein according to the present application;
FIG. 11 shows the activation of lymphocytes to secrete IFN-γ, by the bispecific fusion protein according to the present application;
FIG. 12 shows the activation of lymphocytes to secrete IL-2, by the bispecific fusion protein according to the present application;
FIG. 13 shows an ADCC activity assay for the bispecific fusion protein according to the present application; and
FIG. 14 shows the inhibition of colon cancer growth in mice by the bispecific fusion protein according to the present application.

### DETAILED DESCRIPTION OF THE PREFERRED EMBODIMENTS

The embodiments of the invention of the present application will be illustrated by specific examples below. Those skilled in the art can easily understand other advantages and effects of the invention of the present application from the content disclosed in the specification.

### TERMS & DEFINITIONS

In the present application, the term "bispecific" generally refers to the capacity of the fusion protein of the present application to interact with two different ligands. In the present application, the bispecific fusion protein can specifically bind both PD-L1 and VEGF.

In the present application, the term "specific binding" generally refers to measurable and reproducible interaction, such as the binding between a target and an antibody, and the existence of the target can be determined in a heterogeneous population of molecules (including biomolecules). For example, an antibody that specifically binds to a target (which may be an epitope) is an antibody that binds to the target with greater affinity, avidity, easiness, and/or duration than it binds to other targets. In an embodiment, the extent to which an antibody binds to an unrelated target is about 10% less than the extent to which the antibody binds to a target, as measured, for example, by radioimmunoassay (RIA). For example, in the present application, the bispecific fusion protein may bind to PD-L1 and VEGF at a dissociation constant (KD) of <1×10⁻⁷ m or lower. For example, in the present application, the PD-L1 antibody may bind to PD-L1 at a dissociation constant (KD) of <1×10⁻⁷ M or lower. In some embodiments, the antibody specifically binds to an epitope on a protein, the epitope being conserved among proteins of different species. In another embodiment, the specific binding may include but does not require exclusive binding.

In the present application, the term "first binding domain" generally refers to a binding domain capable of specifically binding to PD-L1. For example, the first binding domain may include an antibody specifically binding to PD-L1.

In the present application, the term "second binding domain" generally refers to a binding domain capable of specifically binding to VEGF. For example, the second binding domain comprises an Ig-like domain of VEGFR1 and an Ig-like domain of VEGFR2.

In the present application, the term "PD-L1" generally refers to a programmed death-ligand 1 protein, and functional variants and/or functional fragments thereof. PD-L1 is also known as cluster of differentiation 274 (CD274) or B7 homologue 1 (B7-H1). The PD-L1 may be a protein encoded by a CD274 gene. PD-L1 binds to its receptor, such as programmed death 1 (PD-1), and PD-1 can be expressed in activated T cells, B cells, and macrophages (Ishida et al., 1992 EMBO J, 11: 3887-3395; Okazaki et al., Autoimmune dilated cardiomyopathy in PD-1 receptor-deficient mice. Science, 2001; 291: 319-22). The complexation of PD-L1 to PD-1 can exert an immunosuppressive action by inhibiting T cell proliferation and producing cytokines IL-2 and IFN-γ (Freeman et al., Engagement of PD-1 immunoinhibitory receptor by a novel B7 family member leads to negative regulation of lymphocyte activation, J.Exp.Med. 2000, 192: 1027-1034; Carter et al., PD-l: PD-L inhibitory pathway affects both CD4(+) and CD8(+) T cells and is overcome by IL-2. EurJ. Immunol. 2002, 32: 634-643). The term "PD-L1" may encompass any natural PD-L1 of any vertebrate origin, including mammals, such as primates (for example, humans) and rodents (e.g., mice and rats). The term described encompasses "full-length" unprocessed PD-L1, and any form of PD-L1 arising from cell processing. PD-L1 can exist as a transmembrane protein or as a soluble protein. This term may also encompass naturally occurring variants (for example, splice variants or allele variants) of PD-L1. The basic structure of PD-L1 may comprise 4 domains: an extracellular Ig-like V-type domain, an Ig-like C2-type domain, a transmembrane domain, and a cytoplasmic domain. The sequence of PD-L1 is known in the art. For example, the information on human PD-L1 gene (including a genomic DNA sequence) can be found under NCBI Gene ID No.: 29126. For another example, the information on mouse PD-L1 gene (including a genomic DNA sequence) can be found under NCBI Gene ID No.: 60533. For another example, the information on mouse PD-L1 gene (including a genomic DNA sequence) can be found under NCBI Gene ID No.: 102145573. Exemplary full-length amino acid sequences of human PD-L1 proteins can be found under NCBI accession number NP_054862 or Uniprot accession number Q9NZQ7. Exemplary full-length sequences of mouse PD-L1 proteins can be found under NCBI accession number NP_068693 or Uniprot accession number Q9EP73. Exemplary full-length sequences of cynomolgus monkey PD-L1 proteins can be found under NCBI accession number XP_005581836 or Uniprot accession number G7PSE7.

In the present application, the term "antibody" generally refers to an immunoglobulin or a fragment or derivative thereof, and encompasses any polypeptide including an antigen-binding site, whether it is produced in vitro or in vivo. The term includes, but is not limited to, polyclonal, monoclonal, monospecific, multispecific, non-specific, humanized, single-chain, chimeric, synthetic, recombinant, hybrid, mutant and grafted antibodies. A basic 4-chain antibody unit is a heterotetrameric glycoprotein composed of two identical light (L) chains and two identical heavy (H) chains. An IgM antibody is composed of 5 basic heterotetrameric units and another polypeptide called J chain, and contains 10 antigen-binding sites; and an IgA antibody includes 2-5 basic 4-chain units that can be polymerized with the J chain to form a multivalent combination. In the case of IgG, the 4-chain unit is generally of about 150,000 Daltons. Each L chain is linked to the H chain by means of a covalent disulfide bond, and two H chains are linked to each other by means of one or more disulfide bonds depending on the isotype of the H chains. Each of the H and L chains also has a regularly spaced intra-chain disulfide bridged bond. Each H chain has a variable domain (VH) at an N-terminus, followed by three constant domains (CH) for each of α and γ chains and four CH domains for the µ and ε isoforms. Each L chain has a variable domain (VL) at its N-terminus and a constant domain at the other end thereof. VL corresponds to VH, and CL corresponds to a first constant domain (CH1) of the heavy chain. Specific amino acid residues are considered to form an interface between the variable domains of the light and heavy chains. VH and VL are paired together to form a single antigen-binding site. For the structures and properties of different types of antibodies, see, for example, Page 71 and Chapter 6 in Basic and Clinical Immunology, 8th Edition, Daniel P. Sties, Abba I. Terr and Tristram G.Parsolw (eds), Appleton & Lange, Norwalk, Conn., 1994. The L chain from any vertebrate species may be classified, based on the amino acid sequence of its constant domain, into one of two distinct types, called κ and λ. Immunoglobulins may be divided into different classes or isotypes depending on the amino acid sequences of constant domains of their heavy chains (CHs). There are five classes of immunoglobulins, namely, IgA, IgD, IgE, IgG, and IgM, which have heavy chains named α, δ, ε, γ, and µ, respectively. The classes γ and α are further divided into subclasses based on the relatively small differences in CH sequence and function. For example, the following subclasses are expressed in a human: IgG1, IgG2A, IgG2B, IgG3, IgG4, IgA1, and IgK1.

In the present application, the term "CDR" generally refers to a region of an antibody variable domain, with a sequence being highly variable and/or forming a structure defined loop. Generally, an antibody includes six CDRs, three (HCDR1, HCDR2, and HCDR3) in VH, and three (LCDR1, LCDR2, and LCDR3) in VL. In a natural antibody, HCDR3 and LCDR3 show most of the diversity of the six CDRs, and in particular, HCDR3 is considered to play a unique role in endowing the antibody with fine specificity. See, for example, Xu et al, Immunity 13:37-45 (2000); Johnson and Wu, in Methods in Molecular Biology 248:1-25 (Lo, ed., Human Press, Totowa, N.J.,2003).

In the present application, the term "VEGF" generally refers to a vascular endothelial growth factor. VEGF can be involved in the regulation of normal and abnormal vascularization and neovascularization associated with tumors and intraocular conditions (see Ferrara, N. and Davis-Smyth, T., Endocr. Rev. l8, 1997, 4-25, etc.). VEGF can play an important regulatory role in neovascularization during embryonic angiogenesis and in angiogenesis during adulthood. VEGF can promote the growth of tumors. VEGF is a highly conserved homodimeric glycoprotein. VEGF may have six isoforms: VEGF-A, VEGF-B (including VEGF-B167 and VEGF-B186), VEGF-C, VEGF-D, and VEGF-E. In the present application, the VEGF may be a human VEGF.

In the present application, the term "VEGFR1" generally refers to a vascular endothelial growth factor 1. VEGFR1 is a type of VEGFR. VEGFR belongs to the receptor tyrosine kinase superfamily and is a membrane mosaic protein. The extramembrane part of VEGFR has approximately 750 amino acid residues and consists of 7 Ig-like domains structurally similar to immunoglobulins. The second Ig domain in the extramembrane region of VEGFR1 is a ligand-binding region. Different spliceosomes of VEGFR1 can competitively bind to VEGFs (for example, VEGF-A or VEGF-B), thereby preventing the binding of VEGF to VEGFR2.

In the present application, the term "VEGFR2" generally refers to a vascular endothelial growth factor 2. The third Ig domain of VEGFR2 may act on the specificity in the binding to a ligand. VEGFR2 can bind to VEGF-A and VEGF-E.

In the present application, the term "Ig-like domain" generally refers to a structure, that may be responsible for binding to VEGF, in the extracellular region of VEGFR. For example, the first Ig-like domain in the extracellular region of VEGFR2 is an essential site for binding to VEGF; the second and third Ig-like domains are main sites for binding to VEGF; the receptor forms the active form of homodimer by means of the fourth Ig-like domain; and the fifth to seventh Ig-like domains are not closely related to the binding to VEGF. For example, the extracellular region of VEGFR1 has 7 Ig-like domains, which may be responsible for binding to VEGF and promoting vascularization.

In the present application, the term "PLGF" generally refers to a placental growth factor, which can be encoded by a PGF gene. The PLGF can be a member of the VEGF subfamily. The PLGF can be expressed in human umbilical vein endothelial cells (HUVE) and placenta. The PLGF can play a role in the growth and differentiation of trophoblasts. The PLGF can be associated with angiogenesis.

In the present application, the term "directly linked" is opposite to the term "indirectly linked", and the term "directly linked" generally refers to direct linkage. For example, the direct linkage may refer to the situation where substances are directly linked without a spacer therebetween. The spacer may be a linker. For example, the linker may be a peptide linker. The term "indirectly linked" generally refers to the situation where substances are not directly linked. For example, the indirect linkage may refer to the situation where the linkage is conducted via a spacer. For example, an N-terminus of the Ig-like domain of VEGFR1 or an N-terminus of the Ig-like domain of VEGFR2 in the present application may be to C-termini of the antibody heavy chains, respectively. For example, the Ig-like domain of VEGFR1 of the present application may be directly linked to the Ig-like domain of VEGFR2.

In the present application, the term "amino acid mutation" generally refers to the substitution of at least one existing amino acid residue with another different amino acid residue. The substituted amino acid residue may be a "naturally occurring amino acid residue", for example, which may be alanine (Ala), arginine (Arg), asparagine (Asn), aspartic acid (Asp), cysteine (Cys), glutamine (Gln), glutamic acid (Glu), glycine (Gly), histidine (His), isoleucine (Ile): leucine (Leu), lysine (Lys), methionine (Met), phenylalanine (Phe), proline (Pro), serine (Ser), threonine (Thr), tryptophan (Trp), tyrosine (Tyr) and valine (Val). The substituted amino acid residue may also be an amino acid residue existing in a non-natural form, for example, which can be norleucine, ornithine, norvaline, homoserine, aib and other amino acid residue analogues. In the present application, the amino acid substitution may be non-conserved substitution. The non-conserved substitution may comprise changing an amino acid residue in a protein or polypeptide of interest in a non-conserved manner, for example, changing an amino acid residue having certain side chain size or certain property (for example, hydrophilicity) to an amino acid residue having a different side chain size or a different property (for example, hydrophobicity). In the present application, the amino acid substitution may also be conserved substitution. The conserved substitution may comprise changing an amino acid residue in a protein or polypeptide of interest in a conserved manner, for example, changing an amino acid residue having certain side chain size or certain property (for example, hydrophilicity) to an amino acid residue having the same or similar side chain size or the same or similar property (for example, still hydrophilicity). Such a conserved substitution typically has no significant impact on the structure or function of the resulting protein.

In the present application, the term "multimer" generally refers to a molecule formed by the binding of monomers. For example, the multimer may be a polyprotein comprising at least two ingredients that are identical or different in structure. For example, the multimer may be a molecule of two or more polypeptide chains that are covalently or non-covalently associated or are bound by covalent or non-covalent interaction. For example, the multimer may be a dimer or a tetramer.

In the present application, the term "first polypeptide chain" generally refers to a PD-L1 antibody-targeting light chain in the bispecific fusion protein of the present application.

In the present application, the term "second polypeptide chain" generally refers to a polypeptide comprising a PD-L1 antibody-targeting heavy chain in the bispecific fusion protein of the present application, a linker of the present application, and a VEGF-targeting second binding domain of the present application. For example, the second polypeptide chain may consist of the PD-L1 antibody-targeting heavy chain in the bispecific fusion protein of the present application, the linker of the present application, and the VEGF-targeting second binding domain of the present application in sequence from an N-terminus.

In the present application, there is no sequential distinction between the "first" and the "second", and the "second" is only a reference indicating a different content from the "first".

In the present application, the term "nucleic acid molecule" generally refers to nucleotide (for example, which may be in an isolated form), deoxyribonucleotide, or ribonucleotide of any length, or an analog that is isolated from its natural environment or is artificially synthesized.

In the present application, the term "vector" generally refers to a means for the delivery of nucleic acids, whereby a polynucleotide encoding a certain protein can be inserted therein to allow the protein to be expressed. The vector may be transformed, transduced, or transfected into a host cell, such that genetic material elements carried thereby can be expressed in the host cell. For instance, the vector may include: a plasmid; a phagemid; a cosmid; an artificial chromosome, such as a yeast artificial chromosome (YAC), a bacterial artificial chromosome (BAC), or a P1-derived artificial chromosome (PAC); and a bacteriophage, such as a λ phage or an M13 phage, an animal virus or the like. The species of animal viruses used as vectors include retroviruses (including lentiviruses), adenoviruses, adeno-associated viruses, herpesviruses (for example, herpes simplex viruses), poxviruses, baculoviruses, papillomaviruses, and papovaviruses (for example, SV40). A vector may contain a variety of elements that control expression, including a promoter sequence, a transcription initiation sequence, an enhancer sequence, a selection element, and a reporter gene. In addition, the vector may also contain an origin of replication site. The vector may also include components, such as, but not limited only to, a virus particle, a liposome, or a protein coat, that assist the vector in entering the cell.

In the present application, the term "cell" generally refers to a single cell, cell line, or cell culture that may be or has been a recipient of a subject's plasmid or vector, including the nucleic acid molecule of the present invention or the vector of the present invention. The cell may include a progeny of a single cell. Due to natural, occasional, or deliberate mutations, the progeny may not necessarily be exactly the same as an original parent cell (in the form of total DNA complement or in the genome). The cell may include a cell transfected in vitro by using the vector defined in the present application. The cell may be a bacterial cell (for example, *E. coli*), a yeast cell, or an additional eukaryotic cell, such as a COS cell, a Chinese hamster ovary (CHO) cell, a CHO-K1 cell, an LNCAP cell, a HeLa cell, a HEK293 cell, a COS-1 cells, an NS0 cell, a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266 cell, or a human multiple myeloma RPMI8226 cell. In certain embodiments, the cell is a mammalian cell. In certain embodiments, the mammalian cell is ah HEK293 cell.

In the present application, the term "pharmaceutical composition" generally refers to a composition suitable for administration to a patient, for example a human patient. For example, the pharmaceutical composition of the present application may comprise the bispecific fusion protein of the present application, the nucleic acid molecule of the present application, the vector of the present application and/or the cell of the present application, and optionally, a pharmaceutically acceptable adjuvant. In addition, the pharmaceutical composition may further comprise a suitable preparation of one or more (pharmaceutically effective) carriers, stabilizers, excipients, diluents, solubilizers, surfactants, emulsifiers and/or preservatives. In the present application, the acceptable ingredient of the pharmaceutical composition may be nontoxic to a subject at a dose and concentration as used. The form of the pharmaceutical composition of the present application may not be limited to, a liquid, frozen, or lyophilized composition.

In the present application, the term "pharmaceutically acceptable adjuvant" generally refers to any and all solvents, dispersion media, coatings, isotonic agents, absorption retarders and others that are compatible with pharmaceutical administration. Such an adjuvant is generally safe and non-toxic, and is neither biologically nor otherwise undesirable.

In the present application, the term "pharmaceutical molecule" generally refers to a molecule with the desired biological efficacy. The pharmaceutical may be prophylactic or therapeutic. The pharmaceutical molecule may include, but is not limited to: protein molecules, including but not limited to peptides, polypeptides, and proteins (including post-translationally modified proteins, fusion proteins, antibodies, or the like). For example, in the present application, the pharmaceutical molecule may further comprise one or more additional amino acids in addition to the bispecific fusion protein of the present application. For example, the pharmaceutical molecule may further comprise other structures in addition to the bispecific fusion protein of the present application. In the present application, the pharmaceutical molecule may further comprise a small molecule structure, including an inorganic or organic compound. In the present application, the pharmaceutical molecule may further comprise a nucleic acid molecule, including but not limited to a double-stranded or single-stranded DNA, or a double-stranded or single-stranded RNA (such as antisense (molecule), RNAi, or the like), an intron sequence, a triple helix nucleic acid molecule, and an aptamer.

In the present application, the term "comprise" generally refers to the inclusion of explicitly specified features, but not excluding other elements.

In the present application, the term "approximately" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

### DETAILED DESCRIPTION OF THE INVENTION

In one aspect, the present application provides a bispecific fusion protein, comprising a first binding domain and a second binding domain, wherein: the first binding domain comprises an antibody specifically binding to PD-L1, wherein the antibody comprises two antibody light chains and two antibody heavy chains, and the antibody light chains and the antibody heavy chains are linked via disulfide bonds; the second binding domain comprises an Ig-like domain of VEGFR1 and an Ig-like domain of VEGFR2; wherein an N-terminus of the Ig-like domain of VEGFR1 or an N-terminus of the Ig-like domain of VEGFR2 is directly or indirectly linked to C-termini of the antibody heavy chains, respectively.

In the present application, the first binding domain may specifically bind to human PD-L1.

In the present application, the second binding domain may specifically bind to a human VEGF family.

In the present application, the second binding domain may specifically bind to a protein selected from the group consisting of: VEGF-A, VEGF-B, VEGF-C, VEGF-D, and PLGF.

In the present application, the Ig-like domain of VEGFR1 may comprise an amino acid sequence as set forth in SEQ ID NO.: 1.

In the present application, the Ig-like domain of VEGFR2 may comprise an amino acid sequence as set forth in SEQ ID NO.: 2.

In the present application, the Ig-like domain of VEGFR1 may be directly linked to the Ig-like domain of VEGFR2. For example, the second binding domain may comprise the Ig-like domain of VEGFR1 and the Ig-like domain of VEGFR2 from an N-terminus in sequence. For example, the second binding domain may consist of the Ig-like domain of VEGFR1 and the Ig-like domain of VEGFR2 from an N-terminus in sequence. In the present application, the second binding domain may comprise an amino acid sequence as set forth in SEQ ID NO: 28.

In the present application, the indirect linking may be conducted via a linker. For example, in the bispecific fusion protein, the N-terminus of the Ig-like domain of VEGFR1 in the second binding domain may be linked to the C-terminus of the antibody heavy chain via the linker. For example, in the bispecific fusion protein, the N-terminus of the Ig-like domain of VEGFR2 in the second binding domain may be linked to the C-terminus of the antibody heavy chain via the linker.

In the present application, the linker may comprise an amino acid sequence as set forth in SEQ ID NO: 3.

In the present application, any known PD-L1 antibody can be used as the antibody in the first binding domain, as long as it has light chain and heavy chain variable regions that may specifically bind to PD-L1.

In the present application, the antibody light chains each comprise a light chain variable region, which may comprise LCDRs 1-3, with the LCDR1 comprising an amino acid sequence as set forth in SEQ ID NO.: 4 or 10.

In the present application, the LCDR2 may comprise an amino acid sequence as set forth in SEQ ID NO.: 5 or SEQ ID NO.: 11.

In the present application, the LCDR3 may comprise an amino acid sequence as set forth in SEQ ID NO.: 6 or SEQ ID NO.: 12.

In the present application, the antibody light chains each comprise a light chain variable region, which may comprise LCDRs 1-3. The LCDR1 may comprise an amino acid sequence as set forth in SEQ ID NO.: 4; the LCDR2 may comprise an amino acid sequence as set forth in SEQ ID NO.: 5; and the LCDR3 may comprise an amino acid sequence as set forth in SEQ ID NO.: 6. Or, the LCDR1 may comprise an amino acid sequence as set forth in SEQ ID NO.: 10; the LCDR2 may comprise an amino acid sequence as set forth in SEQ ID NO.: 11; and the LCDR3 may comprise an amino acid sequence as set forth in SEQ ID NO.: 12.

In the present application, the antibody light chains may each comprise a light chain variable region, which may comprise an amino acid sequence as set forth in SEQ ID NO.: 16 or 17.

In the present application, the antibody light chains may each comprise a light chain constant region, which is derived from a light chain constant region of a protein selected from the group consisting of: Igx and Igλ.

In the present application, the antibody light chains may each comprise an amino acid sequence as set forth in SEQ ID NO: 23 or 24.

In the present application, the antibody heavy chains may each comprise a heavy chain variable region, which may comprise HCDRs 1-3. The HCDR1 may comprise an amino acid sequence as set forth in SEQ ID NO.: 7 or 13.

In the present application, the HCDR2 may comprise an amino acid sequence as set forth in SEQ ID NO.: 8 or SEQ ID NO.: 14.

In the present application, the HCDR3 may comprise an amino acid sequence as set forth in SEQ ID NO.: 9 or SEQ ID NO.: 15.

In the present application, the antibody heavy chains may each comprise a heavy chain variable region, which may comprise HCDRs 1-3. The HCDR1 may comprise an amino acid sequence as set forth in SEQ ID NO.: 7; the HCDR2 may comprise an amino acid sequence as set forth in SEQ ID NO.: 8; and the HCDR3 may comprise an amino acid sequence as set forth in SEQ ID NO.: 9. Or, the HCDR1 may comprise an amino acid sequence as set forth in SEQ ID NO.: 13; the HCDR2 may comprise an amino acid sequence as set forth in SEQ ID NO.: 14; and the HCDR3 may comprise an amino acid sequence as set forth in SEQ ID NO.: 15.

In the present application, the antibody heavy chains may each comprise a heavy chain variable region, which may comprise an amino acid sequence as set forth in SEQ ID NO.: 18 or 19.

In the present application, the light chain variable region of the antibody may comprise an amino acid sequence as set forth in SEQ ID NO.: 16, and the heavy chain variable region of the antibody may comprise an amino acid sequence as set forth in SEQ ID NO.: 18; or the light chain variable region of the antibody may comprise an amino acid sequence as set forth in SEQ ID NO.: 17, and the heavy chain variable region of the antibody may comprise an amino acid sequence as set forth in SEQ ID NO.: 19.

In the present application, the antibody heavy chain may comprise an Fc region.

In the present application, the Fc region may be derived from an Fc of a protein selected from the group consisting of: IgG1 and IgG4.

In the present application, the Fc region may comprise an amino acid mutation at an amino acid position selected from the group consisting of: N298, D357 and L359.

In the present application, an amino acid mutation at N298 in the Fc region may comprise N298A, i.e., the mutation of an amino acid N at position 82 to an amino acid A in an amino acid sequence as set forth in SEQ ID NO.: 20. In the present application, an amino acid mutation at D357 in the Fc region may comprise D357E, i.e., the mutation of an amino acid D at position 141 to an amino acid E in an amino acid sequence as set forth in SEQ ID NO.: 20. In the present application, an amino acid mutation at L359 in the Fc region may comprise L359M, i.e., the mutation of an amino acid L at position 143 to an amino acid M in an amino acid sequence as set forth in SEQ ID NO.: 20.

In the present application, the Fc region may comprise an amino acid mutation selected from the group consisting of: N298A, D357E, and L359M. In the present application, the Fc region may have an amino acid mutation, which may consist of N298A, D357E and L359M.

In the present application, the Fc region may comprise an amino acid sequence as set forth in any one of SEQ ID NOs.: 20-22.

In the present application, the bispecific fusion protein may be a multimer consisting of two copies of first polypeptide chain and second polypeptide chain, wherein the first polypeptide chain may comprise the antibody light chain, and wherein the second polypeptide chain may comprise the antibody heavy chain, the linker and the second binding domain from the N-terminus in sequence.

In the present application, the first polypeptide chain may consist of the antibody light chains. For example, the first polypeptide chains and the antibody heavy chains in the second polypeptide chain may be linked via covalent bonds (for example, disulfide bonds); and the first polypeptide chains and the antibody heavy chains in the second polypeptide chain may be linked via non-covalent bonds.

In the present application, the two first polypeptide chains may comprise the same amino acid sequences. For example, the two first polypeptide chains may have the same amino acid sequences.

In the present application, the two second polypeptide chains may comprise the same amino acid sequences. For example, the two second polypeptide chains may have the same amino acid sequence.

The two first polypeptide chains may be arbitrarily linked to the two second polypeptide chains respectively via a covalent bond and/or non-covalent bond, such that the bispecific fusion protein of the present application can be formed.

In the present application, the first polypeptide chains may each comprise an amino acid sequence as set forth in any one of SEQ ID NOs.: 23-24.

In the present application, the second polypeptide chains may each comprise an amino acid sequence as set forth in any one of SEQ ID NOs.: 25 -27.

In the present application, the first polypeptide chain may comprise an amino acid sequence as set forth in SEQ ID NO.: 23, and the second polypeptide chain may comprise an amino acid sequence as set forth in SEQ ID NO.: 25; or the first polypeptide chain may comprise an amino acid sequence as set forth in SEQ ID NO.: 24, and the second polypeptide chain may comprise an amino acid sequence as set forth in SEQ ID NO.: 26; or the first polypeptide chain may comprise an amino acid sequence as set forth in SEQ ID NO.: 23, and the second polypeptide chain may comprise an amino acid sequence as set forth in SEQ ID NO.: 28.

In the present application, the amino acid sequence of the first polypeptide chain may be as set forth in SEQ ID NO: 23; and the amino acid sequence of the second polypeptide chain may be as set forth in SEQ ID NO: 25. In the present application, the amino acid sequence of the first polypeptide chain may be as set forth in SEQ ID NO: 24; and the amino acid sequence of the second polypeptide chain may be as set forth in SEQ ID NO: 26. Or, in the present application, the amino acid sequence of each of the first polypeptide chains may be as set forth in SEQ ID NO: 23; and the amino acid sequence of each of the second polypeptide chains may be as set forth in SEQ ID NO: 28.

In another aspect, the present application provides a polynucleotide encoding the bispecific fusion protein of the present application.

The polynucleotide of the present application may be isolated. For example, it can be produced or synthesized by the following methods: (i) *in vitro* amplification, for example by polymerase chain reaction (PCR) amplification, (ii) clonal recombination, (iii) purification, for example, by fractionation through restriction digestion and gel electrophoresis, or (iv) synthesis, for example, by chemical synthesis. In some embodiments, the isolated nucleic acid(s) is/are a nucleic acid molecule(s) prepared by the recombinant DNA technology. Recombinant DNA and molecular cloning techniques include those techniques described by Maniatis, T., Fritsch, E.F., Sambrook, J.: Molecular Cloning. A laboratory manual. Cold Spring Harbour Laboratory. Cold Spring Harbour, NY,1982; T. J. Silhavy, M. L. Bennan and L. W. Enquist, Experiments with Gene Fusions, Cold Spring Harbor Laboratory, Cold Spring Harbor, N.Y. (1984); and Ausubel, F. M. et al., Current Protocols in Molecular Biology, pub. By Greene Publishing Assoc, and Wiley-Interscience (1987). Briefly, the nucleic acid may be prepared from genomic DNA fragments, cDNAs, and RNAs, and all these nucleic acids may be extracted directly from cells or recombinantly produced by various amplification methods (including but not limited to PCR and RT-PCR).

The polynucleotide of the present application may comprise at least two different nucleotide sequences. The polynucleotides of the present application may encode at least two different components of the bispecific fusion protein of the present application. For example, the polynucleotide may encode the first polypeptide chain of the present application, and/or, the polynucleotide may encode the second polypeptide chain of the present application. For example, the polynucleotide may encode the light chain of the PD-L1 antibody; the heavy chain of the PD-L1 antibody; the second binding domain; the linker; the Ig-like domain of VEGFR1; and/or the Ig-like domain of VEGFR2.

In another aspect, the present application provides a vector, comprising the polynucleotide of the present application.

In the present application, the vector may comprise one or more polynucleotides of the present application. For example, the vector may be used directly to express the bispecific fusion protein of the present application. In the present application, the vector may be used to express any component of the bispecific fusion protein of the present application. In the present application, at least 2 of the vectors may be used to express the bispecific fusion protein of the present application. For example, the vector may express the first polypeptide chain of the present application, and/or, the vector may express the second polypeptide chain of the present application. For example, the vector may express the light chain of the PD-L1 antibody, the heavy chain of the PD-L1 antibody, the linker, the Ig-like domain of VEGFR1, and/or the Ig-like domain of VEGFR2.

In the present application, the vector may further comprise an additional gene, for example, a marker gene that is allowed to select this vector in a suitable host cell and under a suitable condition. In addition, the vector may further comprise an expression control element that allows a coding region to be expressed correctly in a suitable host. Such a control element is well known to those skilled in the art, and may include, for example, a promoter, a ribosome binding site, an enhancer, and other control elements that regulate gene transcription or mRNA translation. In some embodiments, the expression control sequence is a regulatable element. A specific structure of the expression control sequence may vary depending on the function of the species or cell type, but may generally include a 5' non-transcribed sequence and 5' and 3' non-translated sequences, for example, a TATA box, a capped sequence, a CAAT sequence, etc., which are involved in transcription and translation initiation, respectively. For example, the 5' non-transcribed expression control sequence may comprise a promoter region, which may comprise a promoter sequence for functionally linking the nucleic acid for transcriptional control. The expression control sequence may further comprise an enhancer sequence or an upstream activator sequence. In the present application, suitable promoters may comprise promoters for SP6, T3, and T7 polymerases, human U6 RNA promoters, CMV promoters, and their artificial hybrid promoters (such as CMV), wherein a moiety of a promoter may be fused with a moiety of a promoter of an additional cellular protein (such as human GAPDH and glyceraldehyde-3-phosphate dehydrogenase) gene, and the promoter may or may not comprise additional introns. The polynucleotide(s) of the present application may be operably linked to the expression control element. The vector may comprise a plasmid, a cosmid, a virus, a bacteriophage, or other vectors commonly used in, for example, genetic engineering. For example, the vector is an expression vector.

In another aspect, the present application provides a cell, comprising or expressing the bispecific fusion protein of the present application, the polynucleotide of the present application or the vector of the present application.

The cell may be a prokaryotic cell (for example, a bacterial cell), a CHO cell, an NS/0 cell, an HEK293T cell or an HEK293A cell, or may be an additional eukaryotic cell, for example, a fungal or yeast cell. The vector of the present application may be introduced into the cell by methods known in the art, for example, electroporation, lipofectine transfection, lipofectamin transfection or the like. For example, the host cell may be COS, CHO, NSO, sf9, sf21, DH5a, BL21 (DE3), or TG1.

In another aspect, the present application provides a method for preparing the bispecific fusion protein of the present application, comprising culturing the cell of the present application under a condition suitable for expressing the bispecific fusion protein of the present application.

For example, an appropriate medium, an appropriate temperature, a culture time and the like may be used, and these methods are understood by those of ordinary skills in the art. In some cases, the method may further comprise the step of harvesting (for example, isolating and/or purifying) the bispecific fusion protein of the present application. For example, affinity chromatography may be conducted with protein G-agarose or protein A-agarose, and the bispecific fusion protein of the present application can be purified and separated by, for example, gel electrophoresis and/or high-performance liquid chromatography.

In another aspect, the present application provides a pharmaceutical composition, comprising the bispecific fusion protein of the present application and a pharmaceutically acceptable carrier.

The pharmaceutically acceptable carrier may comprise a buffer, an antioxidant, a preservative, a low molecular weight polypeptide, a protein, a hydrophilic polymer, an amino acid, a sugar, a chelating agent, a counterion, a metal complex and/or a non-ionic surfactant, etc.

In the present application, the pharmaceutical composition may be formulated for oral administration, intravenous administration, intramuscular administration, in situ administration at a tumor site, inhalation, rectal administration, vaginal administration, transdermal administration, or administration via a subcutaneous depot. For example, for oral administration, the pharmaceutical composition may be prepared into small tablets, tablets, capsules, elixirs, suspensions, syrups or flakes. For injectable formulations, the pharmaceutical composition may be prepared into, for example, an ampoule in a dosage form of single dose, or for example, a unit dosage form in a multidose container. The pharmaceutical composition may also be prepared into solutions, suspensions, tablets, pills, capsules and long-acting formulations.

The administration frequency and dose of the pharmaceutical composition may be determined by a number of relevant factors, including the type of disease to be treated, the route of administration, the patient's age, gender, weight and severity of the disease, as well as the type of a medicament as the active ingredient. Due to the excellent in vivo efficacy and concentration duration of the pharmaceutical composition, it can significantly reduce the administration frequency and dose of the medicament.

In another aspect, the present application provides a pharmaceutical molecule, comprising the bispecific fusion protein as defined. In the present application, the pharmaceutical molecule may have the properties and/or functions of the bispecific fusion protein. In the present application, the pharmaceutical molecule may also have other properties and/or functions. For example, the pharmaceutical molecule may further comprise an additional structure. For example, the pharmaceutical molecule may further comprise one or more other amino acids.

In another aspect, the application provides use of the bispecific fusion protein of the present application in preparation of a medicament for treating diseases, wherein the diseases comprise tumors.

The bispecific fusion protein of the present application, and/or the pharmaceutical composition of the present application, are/is used for treating diseases. wherein the diseases comprise tumors.

The present application provides a method for treating diseases, comprising administering an effective amount of a bispecific fusion protein, and/or the pharmaceutical composition of the present application, to a subject in need thereof, where the diseases comprise tumors.

In the present application, the tumors may be solid tumors or non-solid tumors. For example, the tumors may comprise various tumor types known to those skilled in the art, and are not limited to one or more specific tumor types. For example, the tumors may comprise a lung cancer, a colorectal cancer, a cervical cancer, a liver cancer, a gastric cancer and/or a renal cancer. For example, the tumors may comprise the colorectal cancer.

In the present application, the tumors may be PD-L1-positive (for example, PD-L1-overexpressed) tumors. In some cases, the tumors may also be PD-L1-negative. In the present application, the tumors may be associated with overexpression of VEGF (for example, in blood vessels).

In another aspect, the present application provides a method for inhibiting angiogenesis (for example, human angiogenesis), comprising administering an effective amount of the bispecific fusion protein of the present application, and/or the pharmaceutical composition of the present application.

In another aspect, the present application provides a method for inhibiting an activity of a VEGF receptor ligand, comprising administering an effective amount of the bispecific fusion protein of the present application, and/or the pharmaceutical composition of the present application.

For example, the activity of the VEGF receptor ligand may comprise the biological activity and/or function of VEGF and/or VEGFR per se. For example, it may comprise the binding of VEGF to VEGFR.

In another aspect, the present application provides a method for inhibiting PD-L1 activity, comprising administering an effective amount of the bispecific fusion protein of the present application, and/or the pharmaceutical composition of the present application.

For example, the PD-L1 activity may comprise the biological activity and/or function of PD-L1 and/or PD-1 per se. For example, it may comprise the binding of PD-L1 to PD-1.

Not wishing to be bound by any particular theory, the following examples are merely to illustrate the technical solutions of the invention of the present application, and are not intended to limit the scope of the invention of the present application.

### Examples

The codes of the proteins involved in the present application have the following meanings:
SG1201: a PD-L1 antibody 1, its light chain variable region has an amino acid sequence as set forth in SEQ ID NO.: 16; and its heavy chain variable region has an amino acid sequence as set forth in SEQ ID NO.: 18.
SG1202: a PD-L2 antibody 1, its light chain variable region has an amino acid sequence as set forth in SEQ ID NO.: 17; and its heavy chain variable region has an amino acid sequence as set forth in SEQ ID NO.: 19.
SG1501: VEGFRs (with an amino acid sequence as set forth in SEQ ID NO.: 28)-Fc (with an amino acid sequence as set forth in SEQ ID NO.: 20) fusion protein;
12VF1: it is the bispecific fusion protein of the present application, with a first polypeptide chain having an amino acid sequence as set forth in SEQ ID NO.: 23, and with a second polypeptide chain having an amino acid sequence as set forth in SEQ ID NO.: 25;
12VF2: it is the bispecific fusion protein of the present application, with a first polypeptide chain having an amino acid sequence as set forth in SEQ ID NO.: 24, and with a second polypeptide chain having an amino acid sequence as set forth in SEQ ID NO.: 26; and
12VF8: it is the bispecific fusion protein of the present application, with a first polypeptide chain having an amino acid sequence as set forth in SEQ ID NO.: 23, and with a second polypeptide chain having an amino acid sequence as set forth in SEQ ID NO.: 27.

### Example 1: Construction of Fusion Protein

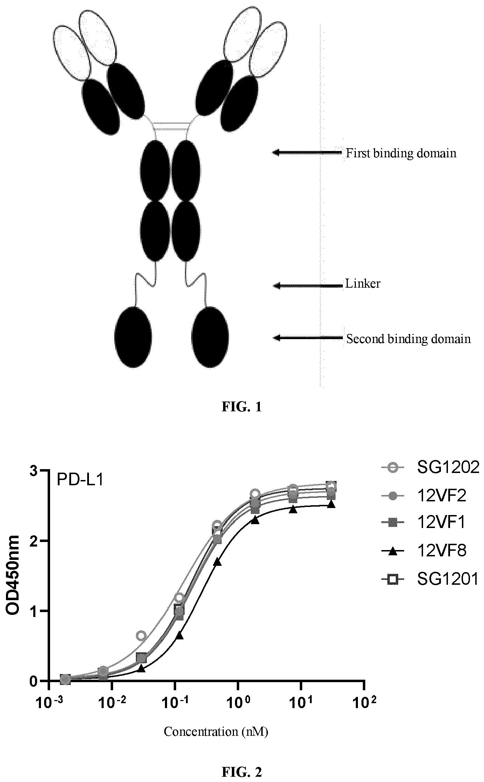

Referring to the structure of the bispecific fusion protein shown in FIG. 1, the sequence of a PD-L1 antibody 1, a linker (with an amino acid sequence as set forth in SEQ ID NO.: 3) and VEGFRs were linked in sequence, where the N-termini of the VEGFRs were linked to the C-terminus of a heavy chain of the PD-L1 antibody 1 to obtain a bispecific fusion protein 12VF1.

On the basis of 12VF1, a mutant IgG1 Fc (with an amino acid sequence as set forth in SEQ ID NO.: 22) was substituted with IgG4 Fc (with an amino acid sequence as set forth in SEQ ID NO.: 21) to obtain a bispecific fusion protein 12VF8.

Referring to the structure of the bispecific fusion protein shown in FIG. 1, a PD-L1 antibody 2, a linker (with an amino acid sequence as set forth in SEQ ID NO.: 3) and VEGFRs were linked in sequence, where the N-termini of the VEGFRs were linked to the C-terminus of a heavy chain of the PD-L1 antibody 2 to obtain a bispecific fusion protein 12VF2.

### Embodiment 2: Activity Assay for Binding to Double Antigens

(1) The binding activity of the bifunctional fusion protein to PD-L1 was evaluated by ELISA using the PD-L1 antibody as a control.

PD-L1 (a human recombinant PD-L1 protein (ECD, His Tag), available from Sino Biological) was used to coat ELISA strips, which were left at 4°C overnight; after PBST washing, 10% fetal bovine serum was added, and blockage was conducted at 37°C for 1 h; antibodies SG1201 and SG1202 of different concentrations and the bispecific fusion proteins 12VF1, 12VF2 and 12VF8 prepared in Example 1 were added to allow reaction at 37°C for 1 h; after PBST washing, horseradish peroxidase-labeled goat anti-human IgG (Fab') 2(HRP) (Abeam) was added to allow reaction at 37°C for 30 min; PBST washing was conducted 5 times; 100 µL of TMB (eBioscience) was added to each well, which was left at room temperature (20±5°C) in the dark for 1-2 min; and then, 100 µL of 2N H₂SO4 stop solution was added to each well to terminate the substrate reaction. The OD value was read at 450 nm by a microplate reader to analyze the binding capacity of the bifunctional fusion protein to PD-L1.

The results are shown in FIG. 2. FIG. 2 showed that the binding capacity of the bispecific fusion protein 12VF1 to PL-L1 is slightly higher than that of 12VF8, and was the same as the antibody SG1201. FIG. 2 showed that the binding capacity of the bispecific fusion protein 12VF2 was comparable to the binding capacity of the antibody SG1202 to PD-L1.

(2) The binding activity of the bifunctional fusion protein to VEGF 165 was evaluated by ELISA using the VEGFR fusion protein as a control.

VEGF165 (Human VEGF165 Protein, His Tag, Aero Biosystems) was used to coat ELISA strips, which were left at 4°C overnight; after PBST washing, 10% fetal bovine serum was added, and blockage was conducted at 37°C for 1 h; bispecific fusion proteins 12VF1, 12VF2, 12VF8 and SG1501 of different concentrations were added to allow reaction at 37°C for 1 h; after PBST washing, horseradish peroxidase-labeled goat anti-human IgG Fc secondary antibody (its horseradish peroxidase (HRP) conjugate, affinity purified, Invitrogen ) was added to allow reaction at 37°C for 30 min; PBST washing was conducted 5 times; 100 µL of TMB (eBioscience) was added to each well, which was left at room temperature (20±5°C) in the dark for 1-2 min; and then, 100 µL of 2N H₂SO4 stop solution was added to each well to terminate the substrate reaction. The OD value was read at 450 nm by a microplate reader to analyze the binding capacities of the bispecific fusion proteins 12VF1, 12VF2 and 12VF8 to VEGF165.

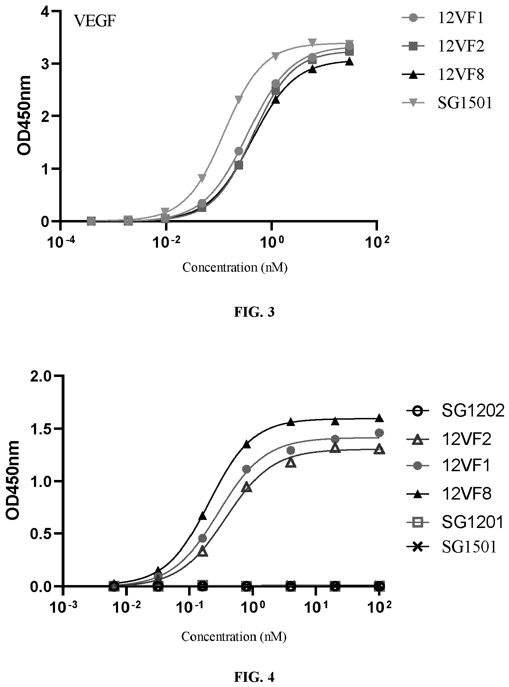

The results were shown in FIG. 3. FIG. 3 showed that the binding capacities of the bispecific fusion proteins 12VF1, 12VF2, and 12VF8 to VEGF165 were comparable, and were slightly weaker than that of the fusion protein SG1501.

### Embodiment 3: Activity Assay for Simultaneous Binding to Double Antigens

The biological activity of the bispecific fusion proteins 12VF1, 12VF2 and 12VF8 prepared in Example 1 for simultaneously binding to double antigens was evaluated by ELISA using the PD-L1 antibodies and VEGFR fusion protein as controls.

PD-L1 (a human recombinant PD-L1 protein (ECD, His Tag), available from Sino Biological) was used to coat ELISA strips, which were left at 4°C overnight; after PBST washing, 10% fetal bovine serum was added, and blockage was conducted at 37°C for 1 h; the bispecific fusion proteins 12VF1, 12VF2 and 12VF8, the pD-L1 antibodies SG1201 and SG1202, and the VEGFR fusion protein SG1501, of different concentrations, were added to allow reaction at 37°C for 1 h; after PBST washing, biotin-labeled VEGF165 (Biotinylated Human VEGF165 Protein, His, Avitag, Aero Biosystems) was added to allow reaction at 37°C for 30 min; PBST washing was conducted 5 times; horseradish peroxidase-labeled avidin (Streptavidin-HRP, Jiaxuan Biotech) was added to allow reaction at 37°C for 30 min; PBST washing was conducted 5 times; 100 µL of TMB (eBioscience) was added to each well, which was left at room temperature (20±5°C) in the dark for 1-2 min; and then, 100 µL of 2N H₂SO4 stop solution was added to each well to terminate the substrate reaction. The OD value was read at 450 nm by a microplate reader to analyze the capacities of the bispecific fusion proteins 12VF1, 12VF2 and 12VF8 to simultaneously binding to VEGF165.

The results were shown in FIG. 4. FIG. 4 showed that the bispecific fusion proteins 12VF1, 12VF2, and 12VF8 were capable of simultaneously binding to PD-L1 and VEGF165, while the PD-L1 antibodies SG1201 and SG1202 and the VEGFRs fusion protein SG1501 were not capable of simultaneously binding to PD-L1 and VEGF165.

### Example 4: Analysis for Activity in Blocking PD-1/PD-L1 Interaction

The biological activity of the bispecific fusion proteins 12VF1, 12VF2 and 12VF8 in blocking the PD-1/PD-L1 interaction was evaluated using the PD-L1 antibodies SG1201 and SG1202 as controls.

PD-L1-his (a human recombinant PD-L1 protein (ECD, His Tag), available from Sino Biological) was used to coat ELISA plates (at l ug/ml), which were left at 4°C overnight; after PBST washing, 10% fetal bovine serum was added, and blockage was conducted at 37°C for 1 h; the bispecific fusion proteins 12VF1, 12VF2 and 12VF8 and the antibodies SG1201 and SG1202, of different concentrations, were added to allow reaction at 37°C for 1 h; after PBST washing, biotin-labeled PD-1 (Biotinylated Human PD-1/PDCD1 Protein, Fc, AvitagTM, HisTag, Aero Biosystems) was added to a final concentration of 2µg/ml, to allow reaction at 37°C for 30 min; PBST washing was conducted 5 times; horseradish peroxidase-labeled avidin (Streptavidin-HRP, Jiaxuan Biotech) was added and then incubated at 37°C for 30 min; PBST washing was conducted 5 times; 100 µL of TMB (eBioscience) was added to each well, which was left at room temperature (20±5°C) in the dark for 1-5 min; and then, 100 µL of 2N H₂SO4 stop solution was added to each well to terminate the substrate reaction. The OD value was read at 450 nm by a microplate reader to analyze the activity of the bispecific fusion proteins 12VF1, 12VF2 and 12VF8 in blocking PD-1/PD-L1.

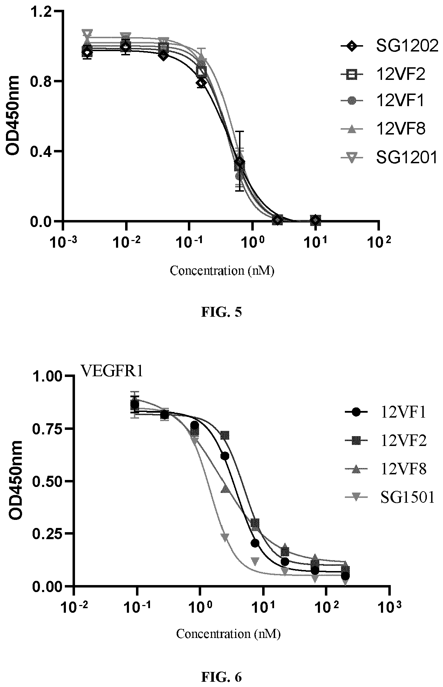

As could be seen from FIG. 5, like the PD-L1 antibodies SG1201 and SG1202, the bispecific fusion proteins 12VF1, 12VF2, and 12VF8 are capable of competitively blocking the binding of PD-1 to PD-L1, with similar activity. Here, the IC₅₀ value of SG1201 was 0.3968 nM, the IC₅₀ value of SG1202 was 0.4216 nM, the IC₅₀ value of 12VF1 was 0.393 nM, the IC₅₀ value of 12VF8 was 0.5002 nM, and the IC₅₀ value of 12VF2 was 0.4256 nM_{∘}

### Example 5: Analysis for Activity in Blocking VEGF/VEGFR Interaction

(1) The biological activity of the bispecific fusion proteins 12VF1, 12VF2 and 12VF8 prepared in Example 1 in blocking the VEGF/VEGFR interaction was evaluated using the fusion protein SG1501 as a control.

VEGFR1 (Human VEGF R1/Flt-1 Protein, His Tag, Aero Biosystems) was used to coat ELISA plates (at l ug/ml), which were left at 4°C overnight; after PBST washing, 10% fetal bovine serum was added, and blockage was conducted at 37°C for 1 h; the bispecific fusion proteins 12VF1, 12VF2 and 12VF8 and the fusion protein SG1501, of different concentrations, were added to allow reaction at 37°C for 1 h; after PBST washing, biotin-labeled VEGF165 (Biotinylated Human VEGF165 Protein, His, Avitag, Aero Biosystems) was added to a final concentration of 0.05 µg/ml and then incubated at 37°C for 30 min; PBST washing was conducted 5 times; horseradish peroxidase-labeled avidin (Streptavidin-HRP, Jiaxuan Biotech) was added and then incubated at 37°C for 30 min; PBST washing was conducted 5 times; 100 µL of TMB (eBioscience) was added to each well, which was left at room temperature (20±5°C) in the dark for 1-5 min; and then, 100 µL of 2N H₂SO4 stop solution was added to each well to terminate the substrate reaction. The OD value was read at 450 nm by a microplate reader to analyze the activity of the bispecific fusion proteins 12VF1, 12VF2 and 12VF8 in blocking VEGF/VEGFR.

The results were shown in FIG. 6, showing that, like the fusion protein SG1501, the bispecific fusion proteins 12VF1, 12VF2, and 12VF8 were capable of competitively blocking the binding of VEGF to its receptor VEGFR1. Here, the IC₅₀ value of 12VF1 was 3.749 nM, the IC₅₀ value of 12VF2 was 5.049 nM, the IC₅₀ value of 12VF8 was 2.182 nM, and the IC₅₀ value of SG1501 was 1.470 nM.

(2) The biological activity of the bispecific fusion proteins 12VF1, 12VF2 and 12VF8 prepared in Example 1 in blocking the VEGF165/VEGFR2 interaction was evaluated using the bispecific fusion protein IMM25011 (see the U.S. Patent Application No. US2020/0172623A1) as a control.

VEGF165 (VEGF165 Protein, Human, Cynomolgus, Recombinant, HPLC-verified, Sino Biological) was used to coat ELISA plates (at l ug/ml), which were left at 4°C overnight; after PBST washing, 10% fetal bovine serum was added, and blockage was conducted at 37°C for 1 h; the bispecific fusion proteins 12VF1, 12VF2, 12VF8 and IMM25011 of different concentrations were added to allow reaction at 37°C for 1 h; after PBST washing, biotin-labeled VEGFR2 (VEGFR2/KDR Protein, Human, Recombinant (His Tag), Biotinylated, Sino Biological) was added to a final concentration of 1 µg/ml to allow reaction at 37°C for 30 min; PBST washing was conducted 5 times; horseradish peroxidase-labeled avidin (Streptavidin-HRP, Jiaxuan Biotech) was added and then incubated at 37°C for 30 min; PBST washing was conducted 5 times; 100 µL of TMB (eBioscience) was added to each well, which was left at room temperature (20±5°C) in the dark for 1-5 min; and then, 100 µL of 2N H₂SO4 stop solution was added to each well to terminate the substrate reaction. The OD value was read at 450 nm by a microplate reader to analyze the activity of the bispecific fusion proteins 12VF1, 12VF2 and 12VF8 in blocking VEGF165/VEGFR2.

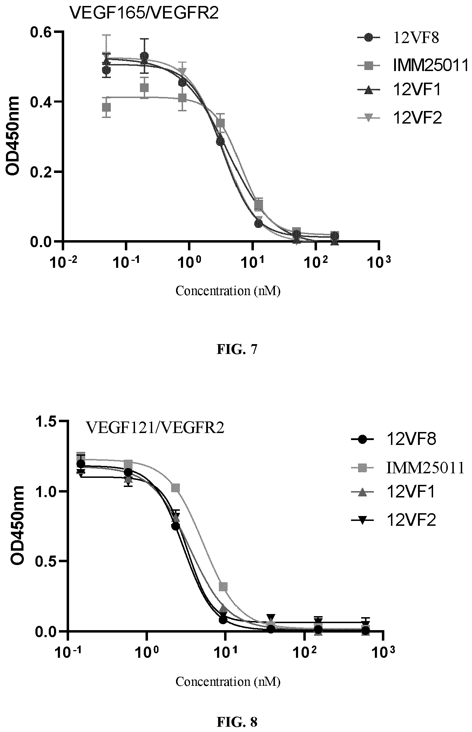

The results were shown in FIG. 7, showing that the bispecific fusion proteins 12VF1, 12VF2, and 12VF8 were capable of blocking the binding of VEGF165 to its receptor VEGFR2, with the activity higher than that of IMM25011. Here, the IC₅₀ value of 12VF1 was 4.092 nM, the IC₅₀ value of 12VF2 was 3.501 nM, the IC₅₀ value of 12VF8 was 3.422 nM, and the IC₅₀ value of IMM25011 was 6.596 nM.

(3) The biological activity of the bispecific fusion proteins 12VF1, 12VF2 and 12VF8 prepared in Example 1 in blocking the VEGF121/VEGFR2 interaction was evaluated using the bispecific fusion protein IMM25011 as a control.

VEGF121 (VEGF 121 Protein, Human, Recombinant, Sino Biological) was used to coat ELISA plates (at l ug/ml), which were left at 4°C overnight; after PBST washing, 10% fetal bovine serum was added, and blockage was conducted at 37°C for 1 h; the bispecific fusion proteins 12VF1, 12VF2, 12VF8 and IMM25011 of different concentrations were added to allow reaction at 37°C for 1 h; after PBST washing, biotin-labeled VEGFR2 (VEGFR2/KDR Protein, Human, Recombinant (HisTag), Biotinylated, Sino Biological) was added to a final concentration of 1 µg/ml to allow reaction at 37°C for 30 min; PBST washing was conducted 5 times; horseradish peroxidase-labeled avidin (Streptavidin-HRP, Jiaxuan Biotech) was added and then incubated at 37°C for 30 min; PBST washing was conducted 5 times; 100 µL of TMB (eBioscience) was added to each well, which was left at room temperature (20±5°C) in the dark for 1-5 min; and then, 100 µL of 2N H2SO4 stop solution was added to each well to terminate the substrate reaction. The OD value was read at 450 nm by a microplate reader to analyze the activity of the bispecific fusion proteins 12VF1, 12VF2 and 12VF8 in blocking VEGF121/VEGFR2.

The results were shown in FIG. 8, showing that the bispecific fusion proteins 12VF1, 12VF2, and 12VF8 were capable of blocking the binding of VEGF121 to its receptor VEGFR2, with the activity higher than that of IMM25011. Here, the IC₅₀ value of 12VF1 was 3.536 nM, the IC₅₀ value of 12VF2 was 3.291 nM, the IC₅₀ value of 12VF8 was 2.955 nM, and the IC₅₀ value of IMM25011 was 5.281 nM.

(4) The biological activity of the bispecific fusion proteins 12VF1, 12VF2 and 12VF8 prepared in Example 1 in blocking the VEGFC/VEGFR2 interaction was evaluated using the bispecific fusion protein IMM25011 as a control.

VEGFC (VEGF C Protein, Human, Recombinant (His Tag), Sino Biological) was used to coat ELISA plates (at l ug/ml), which were left at 4°C overnight; after PBST washing, 10% fetal bovine serum was added, and blockage was conducted at 37°C for 1 h; the bispecific fusion proteins 12VF1, 12VF2, 12VF8 and IMM25011 were each added at 15µM to allow reaction at 37°C for 1 h; after PBST washing, biotin-labeled VEGFR2 (VEGFR2/KDR Protein, Human, Recombinant (HisTag), Biotinylated, Sino Biological) was added to a final concentration of 20 µg/ml to allow reaction at 37°C for 30 min; PBST washing was conducted 5 times; horseradish peroxidase-labeled avidin (Streptavidin-HRP, Jiaxuan Biotech) was added and then incubated at 37°C for 30 min; PBST washing was conducted 5 times; 100 µL of TMB (eBioscience) was added to each well, which was left at room temperature (20±5°C) in the dark for 1-5 min; and then, 100 µL of 2N H₂SO4 stop solution was added to each well to terminate the substrate reaction. The OD value was read at 450 nm by a microplate reader to analyze the activity of the bispecific fusion proteins 12VF1, 12VF2 and 12VF8 in blocking VEGFC/VEGFR2.

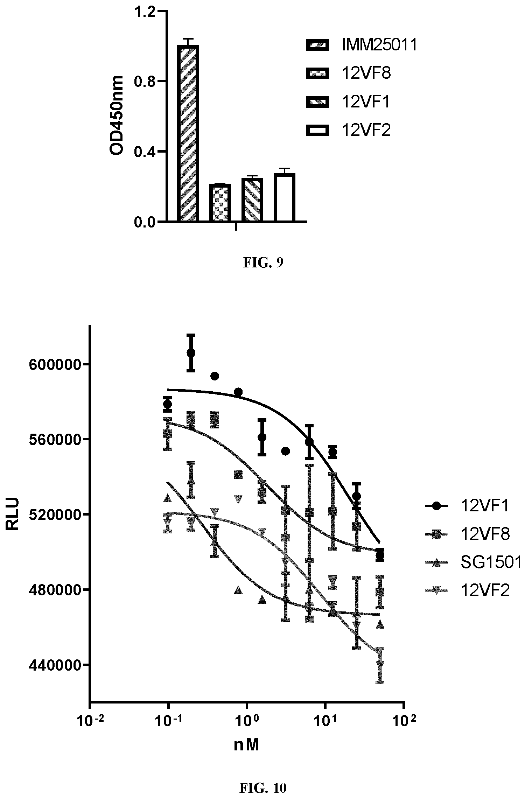

The results were shown in FIG. 9, showing that the bispecific fusion proteins 12VF1, 12VF2, and 12VF8 were capable of blocking the binding of VEGFC to its receptor VEGFR2 to thus effectively inhibit angiogenesis, and 12VF1, 12VF2, and 12VF8 were similar in blocking activity. However, IMM25011 was not capable of blocking the binding of VEGFC to its receptor VEGFR2.

Therefore, compared with IMM25011 that can only block the binding of VEGF121 to VEGFR2, the bispecific fusion proteins of the present application can block the binding of VEGF121 to VEGFR2 and the binding of VEGF C to its receptor VEGFR2, thereby playing a regulatory role of this signaling pathway in a more comprehensive way to take the effect of more completely blocking the angiogenesis at a tumor site.

### Example 6: Analysis of Activity in Inhibiting Proliferation of HUVEC Cells

The biological activity of the bispecific fusion proteins 12VF1, 12VF2 and 12VF8 in inhibiting the proliferation of HUVEC cells was evaluated using a fusion protein SG1501 as a control.

HUVEC cells were resuspended with an experimental medium, then added to a 96-well cell culture plate, and incubated overnight in an incubator; VEGF 165 (ActiveMax^{®} Human VEGF 165 Protein, Tag Free (MALS verified), Aero Biosystems) was diluted to 40 ng/mL with the experimental medium, then isovolumetrically mixed with the bispecific fusion proteins 12VF1, 12VF2 and 12VF8 and fusion protein SG1501 at different concentrations, and pre-incubated at 37°C for 30 min, and a resulting mixture was added to the 96-well cell culture plate; after 3 days of incubation in the incubator at 37°C, the 96-well plate was taken out and equilibrated at room temperature for 10 min; 100 µL of CellTiter-Lumi reagent (CellTiter-LumiTM Luminescent Cell Viability Assay Kit, Beyotime Biotechnology) was added to each well, which was gently shaken and mixed to allow reaction in the dark for 10 min; and then, the relative fluorescence intensity value (RLU) of chemiluminescence was read with a microplate reader to analyze the activity of the bispecific fusion proteins 12VF1, 12VF2 and 12VF8 in inhibiting the proliferation of HUVEC cells.

As could be seen from FIG. 10, the bispecific fusion proteins 12VF1, 12VF2, and 12VF8 were capable of inhibiting the proliferation of HUVEC cells, with the activity slightly weaker than that of the fusion protein SG1501.

### Example 7: Analysis of Activity in Activating T lymphocytes

The biological activity of the bispecific fusion protein 12VF8 in activating T lymphocytes in mixed lymphocyte responses was evaluated using the PD-L1 antibody SG1201 and isotype control antibody as controls.

Monocytes were isolated from PBMCs from a healthy donor with CD14 microbeads (Miltenyi) and were differentiated into dendritic cells under the induction by ImmunoCultTM Dendritic Cell Culture Kit (Stemcell); CD4+ T lymphocytes were isolated from PBMCs from another healthy donor with a CD4+T Cell Isolation Kit (Miltenyi); the CD4+ T lymphocytes and the dendritic cells were added to a 96-well cell culture plate at a ratio of 5:1 in terms of cell count, the bispecific fusion proteins 12VF8 of different concentrations were then added, and they were mixed well and then incubated in an incubator at 37°C for 5 days; cell supernatants were collected; and the concentrations of cytokines IFN-γ and IL-2 were detected using ELISA kits to analyze the activity of the bispecific fusion protein 12VF8 in activating T lymphocytes in the mixed lymphocyte responses.

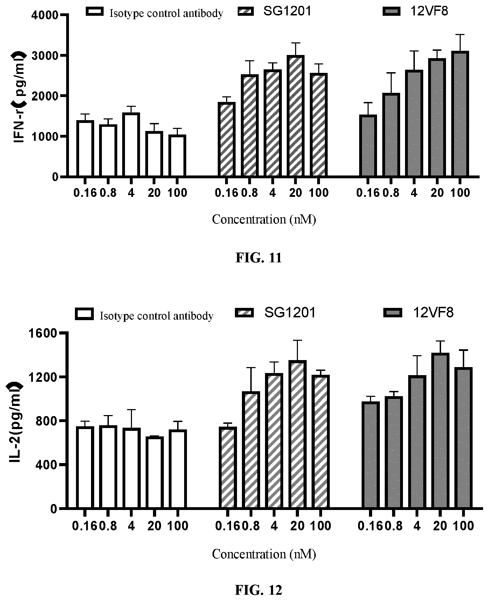

As could be seen from FIGs. 11-12, compared with an isotype control group, the bispecific fusion protein 12VF8 could activate the T cells in a dose-dependent manner just as the PD-L1 antibody SG1201, thereby promoting the release of IFN-γ and IL-2.

### Example 8: Analysis of ADCC Activity

The ADCC activity of the bispecific fusion proteins 12VF1 and 12VF8 prepared in Example 1 was evaluated using a reporter gene method with the bispecific fusion protein IMM25011 as a control, PD-L1-overexpressing cell lines CHO-K1/PD-L1 as target cells, and Jurkat cells overexpressing human FcyRIIIa genes and NFAT fluorescent reporter genes (referred to as Jurkat-ADCC cells) as effector cells.

CHO-K1/PD-L1 cells were collected and added to a 96-well cell culture plate, with 2×10⁴ cells per well; Jurkat-ADCC cells were collected and added to a 96-well cell culture plate, with 1.2×10⁵ cells per tube; the bispecific fusion proteins IMM25011, 12VF1 and 12VF8 of different concentrations were added and then incubated at 37°C for 6 h; 100 µL of luciferase detection solution was added to each well to allow reaction at room temperature in the dark for 10 min; and then, the relative fluorescence intensity value (RLU) of chemiluminescence was read by a microplate reader to analyze the ADCC activity of the bispecific fusion proteins 12VF1 and 12VF8.

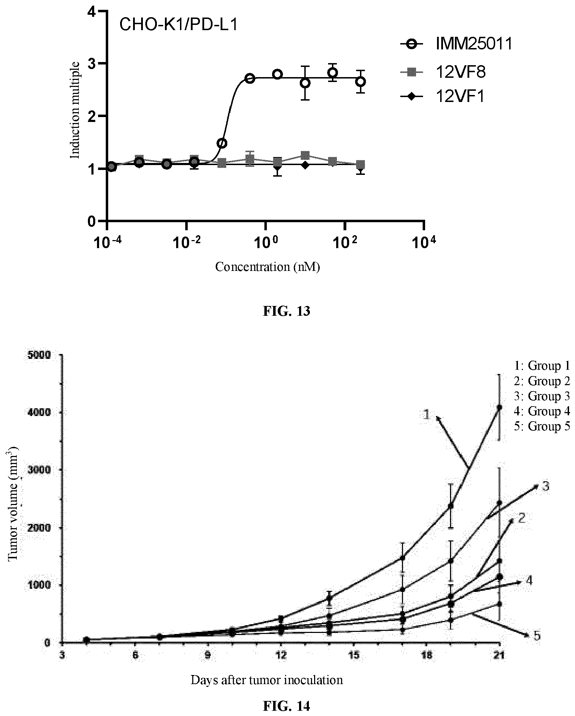

The results were shown in FIG. 13, showing that the bispecific fusion protein IMM25011 had ADCC activity, while 12VF1 and 12VF8 had no ADCC activity.

The above results show that 12VF1 and 12VF8 have better safety. The killing of other normal cells positive to PD-L1 was avoided, thereby reducing the potential toxic and side effects. PD-L1 was also expressed in many normal immune cells, such as myeloid DCs, macrophages, and lymphoid T effector cells, Tregs, and NK cells. Therefore, 12VF1 and 12VF8 could avoid killing normal tissues in the absence of ADCC activity.

### Example 9: Activity of Fusion Proteins in In vivo Inhibition of Tumors

The effect of the bispecific fusion protein 12VF8 on inhibiting tumor activity was evaluated with a mouse colorectal cancer MC38 model.

Mouse colorectal cancer cells MC38 were subcutaneously inoculated to the right anterior flank ribs of male C57BL/6J mice, with 36 mice in total. When the tumors grew to about 58 mm³, group administration was conducted, with 5 groups in total, 6 mice in each group. Specifically, in Group 1: PBS (i.e., as a vehicle group) was administered by intraperitoneal injection on Day 17 of the trial, twice a week for three weeks; Group 2: SG1201 (2 mg/kg) was administered by intraperitoneal injection, twice a week for three weeks; Group 3: SG1501 (1.4 mg/kg), was administered by intraperitoneal injection, twice a week for three weeks; Group 4: SG1201 (2 mg/kg) and SG1501 (1.4 mg/kg) were administered simultaneously by intraperitoneal injection, twice a week for three weeks; and Group 5: 12VF8 (2.7 mg/kg) was administered by intraperitoneal injection, twice a week for three weeks. The results after administration sequentially correspond to Groups 1-5 in FIG. 14. The tumor volume and body weight were measured weekly, and the relationship between the body weight and tumor volume of the tumor-bearing mice and administration time was recorded. The serum was collected from fixed mice in each group 2 hours after the last administration. At the end of the experiment, the tumor-bearing mice were euthanized, and the tumors were isolated, weighed, and photographed. The tumor growth inhibition rate TGITV (%) was calculated and statistically analyzed.

The results showed that at the end of the experiment, the tumor growth inhibition rates of Groups 2, 3, 4, and 5 were 63%, 39%, 71% and 83%, respectively, and the tumor volume in each treatment group was significantly lower than that of Group 1 (p<0.05) as a control, indicating a significant antitumor effect to effectively inhibit the tumor growth. Moreover, the tumor volume handled in Group 5 was the smallest among all treatment groups, and significantly lower than that of Group 3 (p<0.05), showing the tumor inhibition activity better than Group 2 or Group 3 where a single agent was administered, and also better than Group 4 where an agent combination was administered. During the treatment, the tumor-bearing mice showed good tolerance to the treatment of Groups 2-5, and the mice in each group had normal body weight and good general condition, without abnormal behaviors. It could be seen that 12VF8 had administration safety.

The foregoing detailed description is provided by way of explanation and examples, and is not intended to limit the scope of the appended claims. Various variations of the embodiments listed in the present application until now would be obvious to those of ordinary skills in the art, and should be kept within the scope of the appended claims and equivalents thereof.

## Claims

1. A bispecific fusion protein, comprising a first binding domain and a second binding domain, wherein:
said first binding domain comprises an antibody specifically binding to PD-L1, wherein said antibody comprises two antibody light chains and two antibody heavy chains, and said antibody light chains and said antibody heavy chains are linked via disulfide bonds;
said second binding domain comprises an Ig-like domain of VEGFR1 and an Ig-like domain of VEGFR2;
wherein an N-terminus of said Ig-like domain of VEGFR1 or an N-terminus of said Ig-like domain of VEGFR2 is directly or indirectly linked to C-termini of said antibody heavy chains, respectively.

2. The bispecific fusion protein according to claim 1, wherein said first binding domain specifically binds to human PD-L1.

3. The bispecific fusion protein according to any one of claims 1-2, wherein said second binding domain specifically binds to a human VEGF family.

4. The bispecific fusion protein according to anyone of claims 1-3, wherein said second binding domain specifically binds to a protein selected from the group consisting of: VEGF-A, VEGF-B, VEGF-C, VEGF-D, and PLGF.

5. The bispecific fusion protein according to any one of claims 1-4, wherein said Ig-like domain of VEGFR1 comprises an amino acid sequence as set forth in SEQ ID NO.: 1.

6. The bispecific fusion protein according to any one of claims 1-5, wherein said Ig-like domain of VEGFR2 comprises an amino acid sequence as set forth in SEQ ID NO.: 2.

7. The bispecific fusion protein according to any one of claims 1-6, wherein said Ig-like domain of VEGFR1 and said Ig-like domain of VEGFR2 are directly linked.

8. The bispecific fusion protein according to any one of claims 1-7, wherein said second binding domain comprises an amino acid sequence as set forth in SEQ ID NO.: 28.

9. The bispecific fusion protein according to any one of claims 1-8, wherein said indirect linking is conducted via a linker.

10. The bispecific fusion protein according to claim 9, wherein said linker comprises an amino acid sequence as set forth in SEQ ID NO.: 3.

11. The bispecific fusion protein according to any one of claims 1-10, wherein said antibody light chains each comprise a light chain variable region, which comprises LCDRs 1-3, with said LCDR1 comprising an amino acid sequence as set forth in any one of SEQ ID NOs.: 4 and 10.

12. The bispecific fusion protein according to claim 11, wherein said LCDR2 comprises an amino acid sequence as set forth in any one of SEQ ID NOs.: 5 and 11.

13. The bispecific fusion protein according to any one of claims 11-12, wherein said LCDR3 comprises an amino acid sequence as set forth in any one of SEQ ID NOs.: 6 and 12.

14. The bispecific fusion protein according to any one of claims 11-13, wherein amino acid sequences of said LCDRs 1-3 are selected from any one of the following groups:
(1) said LCDR1 comprises an amino acid sequence as set forth in SEQ ID NO.: 4, said LCDR2 comprises an amino acid sequence as set forth in SEQ ID NO.: 5, and said LCDR3 comprises an amino acid sequence as set forth in SEQ ID NO.: 6; and
(2) said LCDR1 comprises an amino acid sequence as set forth in SEQ ID NO.: 10, said LCDR2 comprises an amino acid sequence as set forth in SEQ ID NO.: 11, and said LCDR3 comprises an amino acid sequence as set forth in SEQ ID NO.: 12.

15. The bispecific fusion protein according to any one of claims 1-14, wherein said antibody light chains each comprise a light chain variable region, which comprises an amino acid sequence as set forth in any one of SEQ ID NOs.: 16-17.

16. The bispecific fusion protein according to any one of claims 1-15, wherein said antibody light chains each comprise a light chain constant region, which is derived from a light chain constant region of a protein selected from the group consisting of: Igx and Igλ.

17. The bispecific fusion protein according to any one of claims 1-16, wherein said antibody heavy chains each comprise a heavy chain variable region, which comprises HCDRs 1-3, with said HCDR1 comprising an amino acid sequence as set forth in any one of SEQ ID NOs.: 7 and 13.

18. The bispecific fusion protein according to claim 17, wherein said HCDR2 comprises an amino acid sequence as set forth in any one of SEQ ID NOs.: 8 and 14.

19. The bispecific fusion protein according to any one of claims 17-18, wherein said HCDR3 comprises an amino acid sequence as set forth in any one of SEQ ID NOs.: 9 and 15.

20. The bispecific fusion protein according to any one of claims 17-19, wherein said HCDRs 1-3 comprise amino acid sequences selected from the following groups:
(1) said HCDR1 comprises an amino acid sequence as set forth in SEQ ID NO.: 7, said HCDR2 comprises an amino acid sequence as set forth in SEQ ID NO.: 8, and said HCDR3 comprises an amino acid sequence as set forth in SEQ ID NO.: 9; and
(2) said HCDR1 comprises an amino acid sequence as set forth in SEQ ID NO.: 13, said HCDR2 comprises an amino acid sequence as set forth in SEQ ID NO.: 14, and said HCDR3 comprises an amino acid sequence as set forth in SEQ ID NO.: 15.

21. The bispecific fusion protein according to any one of claims 1-20, wherein said antibody heavy chains each comprise a heavy chain constant region, which comprises an amino acid sequence as set forth in any one of SEQ ID NOs.: 18-19.

22. The bispecific fusion protein according to any one of claims 1-21, wherein said antibody heavy chains each comprises an Fc region.

23. The bispecific fusion protein according to claim 22, wherein said Fc region is derived from an Fc of a protein selected from the group consisting of: IgG1 and IgG4.

24. The bispecific fusion protein according to any one of claims 22-23, wherein said Fc region comprises an amino acid mutation at an amino acid position selected from the group consisting of: N298, D357 and L359.

25. The bispecific fusion protein according to any one of claims 22-24, wherein said Fc region comprises an amino acid mutation selected from the group consisting of: N298A, D357E and L359M.

26. The bispecific fusion protein according to any one of claims 22-25, wherein said Fc region comprises an amino acid sequence as set forth in any one of SEQ ID NOs.: 20-22.

27. The bispecific fusion protein according to any one of claims 9-26, which is a multimer consisting of two copies of first polypeptide chain and second polypeptide chain,
wherein said first polypeptide chain comprises said antibody light chains, and
wherein said second polypeptide chain comprises said antibody heavy chains, said linker and said second binding domain in sequence from an N-terminus.

28. The bispecific fusion protein according to claim 27, wherein said first polypeptide chain comprises an amino acid sequence as set forth in any one of SEQ ID NOs.: 23-24.

29. The bispecific fusion protein according to any one of claims 27-28, wherein said second polypeptide chain comprises an amino acid sequence as set forth in any one of SEQ ID NOs.: 25-27.

30. The bispecific fusion protein according to any one of claims 27-29, wherein said first polypeptide chain comprises an amino acid sequence as set forth in SEQ ID NO.: 23, and said second polypeptide chain comprises an amino acid sequence as set forth in SEQ ID NO.: 25; or
wherein said first polypeptide chain comprises an amino acid sequence as set forth in SEQ ID NO.: 24, and said second polypeptide chain comprises an amino acid sequence as set forth in SEQ ID NO.: 26; or
wherein said first polypeptide chain comprises an amino acid sequence as set forth in SEQ ID NO.: 23, said second polypeptide chain comprises an amino acid sequence as set forth in SEQ ID NO.: 27.

31. A polynucleotide encoding the bispecific fusion protein of any one of claims 1-30.

32. A vector, comprising the polynucleotide of claim 31.

33. A cell, comprising the vector of claim 32.

34. A method for preparing the bispecific fusion protein of any one of claims 1-30, comprising culturing the cell of claim 33 under a condition suitable for expressing the bispecific fusion protein of any one of claims 1 to 30.

35. A pharmaceutical composition, comprising the bispecific fusion protein of any one of claims 1-30 and an optionally pharmaceutically acceptable carrier.

36. A pharmaceutical molecule, comprising the bispecific fusion protein of any one of claims 1-30.

37. Use of the bispecific fusion protein according to any one of claims 1-30, the polynucleotide of claim 31, the vector of claim 32, the cell of claim 33, the pharmaceutical composition of claim 35, and/or the pharmaceutical molecule of claim 36 in preparation of a medicament for treating diseases, wherein said diseases comprise tumors.

38. The use according to claim 37, wherein said diseases comprise solid tumors and non-solid tumors.

39. The use according to any one of claims 37-38, wherein said diseases comprise PD-L1-positive tumors.

40. The use according to any one of claims 37-39, wherein said tumors comprise a lung cancer, a colorectal cancer, a cervical cancer, a liver cancer, a gastric cancer and/or a renal cancer.

41. A method for inhibiting angiogenesis, comprising administering an effective amount of the bispecific fusion protein according to any one of claims 1-30, the polynucleotide of claim 31, the vector of claim 32, the cell of claim 33, the pharmaceutical composition of claim 35, and/or the pharmaceutical molecule of claim 36.

42. A method for inhibiting an activity of a VEGF receptor ligand, comprising administering an effective amount of the bispecific fusion protein according to any one of claims 1-30, the polynucleotide of claim 31, the vector of claim 32, the cell of claim 33, the pharmaceutical composition of claim 35, and/or the pharmaceutical molecule of claim 36.

43. A method for inhibiting of PD-L1 activity, comprising administering an effective amount of the bispecific fusion protein according to any one of claims 1-30, the polynucleotide of claim 31, the vector of claim 32, the cell of claim 33, the pharmaceutical composition of claim 35, and/or the pharmaceutical molecule of claim 36.
